Europäisches Patentamt

European Patent Office

Office européen des brevets

(11) Publication number: **0 320 239 B1**

(12) **EUROPEAN PATENT SPECIFICATION**

(45) Date of publication of patent specification: **05.08.92**  (51) Int. Cl.5: **A01G 7/00**

(21) Application number: **88311592.5**

(22) Date of filing: **07.12.88**

(54) **Method of preparation of plant tissue strips for plant tissue culture.**

(30) Priority: **08.12.87 JP 311196/87**
**30.04.88 JP 108029/88**
**23.06.88 JP 153586/88**
**31.10.88 JP 276530/88**
**01.11.88 JP 278456/88**

(43) Date of publication of application:
**14.06.89 Bulletin 89/24**

(45) Publication of the grant of the patent:
**05.08.92 Bulletin 92/32**

(84) Designated Contracting States:
**AT BE CH DE ES FR GB GR IT LI LU NL SE**

(56) References cited:
**EP-A- 0 132 414**     **EP-A- 0 232 628**
**EP-A- 0 298 722**     **EP-A- 0 300 827**
**FR-A- 1 318 483**     **FR-A- 2 443 907**
**US-A- 4 350 768**

(73) Proprietor: **MITSUI PETROCHEMICAL INDUS-TRIES, LTD.**
**2-5, Kasumigaseki 3-chome Chiyoda-ku Tokyo 100(JP)**

(72) Inventor: **Kawarbayashi, Waichirou**
**MITSUI PETROCHEMICAL IND. LTD. 6-1-2, Waki**

Waki-cho Kuga-gun Yamaguchi(JP)
Inventor: **Maubara, Kouichi**
**MITSUI PETROCHEMICAL IND. LTD. 6-1-2, Waki**
Waki-cho Kuga-gun Yamaguchi(JP)
Inventor: **Yamagata, Hikaru**
**MITSUI PETROCHEMICAL IND. LTD. 6-1-2, Waki**
Waki-cho Kuga-gun Yamaguchi(JP)
Inventor: **Takahashi, Shigeru**
**MITSUI PETROCHEMICAL IND. LTD. 6-1-2, Waki**
Waki-cho Kuga-gun Yamaguchi(JP)
Inventor: **Motoyama, Yoshio**
**MITSUI PETROCHEMICAL IND. LTD. 6-1-2, Waki**
Waki-cho Kuga-gun Yamaguchi(JP)
Inventor: **Hirata, Yukimasa**
**MITSUI PETROCHEMICAL IND. LTD. 6-1-2, Waki**
Waki-cho Kuga-gun Yamaguchi(JP)
Inventor: **Shirane, Yoshiko**
**MITSUI PETROCHEMICAL IND. LTD. 6-1-2, Waki**
Waki-cho Kuga-gun Yamaguchi(JP)

(74) Representative: **Myerscough, Philip Boyd et al
J.A.Kemp & Co. 14, South Square Gray's Inn
London, WC1R 5EU(GB)**

## Description

### 1. Field of the invention

The present invention relates to a method of preparation of a large amount of plant tissue strips which can be regenerated to plants in plant tissue culture.

### 2. Description of the Related Art

Conventionally, sections of plant organs or tissues used in plant tissue culture are prepared manually, for example, by slicing plant material with a scalpel or cutting plant discs from a plant material with a cork borer. These methods are advantageous in that the operator can select a part of a plant material most suitable for the culture, and can cut the plant material into a shape most preferable for the culture. Nevertheless, the operation requires a high level of skill and is labor and time-consuming. Therefore, although such conventional methods can be used for experimental activities in a laboratory, it is difficult to apply such methods to the preparation of plant tissue strips for commercial proliferation of plants.

Although EP No. 132414A describes a semi-automated plant tissue culturing, it does not suggest a particular cutting apparatus similar to that of the present invention.

### SUMMARY OF THE INVENTION

The present inventors surprisingly found that, by selecting a method of cutting a plant material appropriate to the kind of plant material to be cut, mechanical cutting will provide sections which can be successfully grown without problems or difficulty.

Therefore, according to the present invention, there is provided a method of preparation of a plurality of plant tissue strips which can regenerate to plants in plant tissue culture, characterized by:

preparing a sterilized or uncontaminated plant material, and

supplying the plant material to a cutting apparatus to prepare plant strips, wherein the cutting apparatus comprises a circular plate 3 or 53 having one or more than one long cutout 4 or 54 positioned at an angle to a tangential line of the circular plate 3 or 53, which plate is rotated by a drive unit 2 or 52, and one more than one blade 5 or 54 fixed to the circular plate 3 or 53 so that a cutting edge 6 of the blade 5 or 55 is positioned above the cutout 4 or 54 with a space between the edge 6 and a plane extending from a surface of the circular plate in the proximity of the blade.

Furthermore, the present invention provides a method of preparation of a plurality of plant tissue strips which can be regenerated to plants in plant tissue culture, characterized by:

preparing a liquid medium in which pieces of a sterilized or uncontaminated plant material are contained, and cutting the pieces of the plant using the above-mentioned apparatus inserted in a conduit through which the liquid medium flows.

### BRIEF DESCRIPTION OF THE DRAWINGS

Figure 1 is a vertical cross-sectional view of an embodiment of the apparatus used in the present method;

Fig. 2 is a horizontal cross-sectional view of the same embodiment as Fig. 1;

Fig. 3 is a cross-sectional view taken along the line A-A in Fig. 2;

Fig. 4 is a cross-sectional view of an embodiment of the apparatus used in the present method;

Fig. 5 is a plane view of the embodiment shown in Fig. 4; and

Fig. 6 is a cross-sectional view taken along the line B-B in Fig. 5.

Fig. 7 is a graph showing a result of cutting a potato having a cylindrical shape, using an apparatus shown in Fig. 1, representing the relationship between the rotation rate and the thickness of the resulting strips and the dispersion thereof; and,

Fig. 8 is a graph similar to that of Fig. 7 except that Fig. 8 represents the relationship between the weight applied to the potato and the thickness of the resulting strips and the dispersion thereof.

### DESCRIPTION OF THE PREFERRED EMBODIMENTS

According to the present invention, a large amount of tissue strips of various kinds of plants can be prepared. Namely, various organs or parts of plants including bulbs, tubers, stems and the like (hereinafter referred to as "plant material") can be sectional by the present methods. The plant material may be obtained from a plant which has not been contaminated with plant pathogens such as bacteria or fungi, such as a plant cultured under aseptic conditions, or the plant material can be sterilised by any conventional method which does not destroy the viability of the plant, for example, a treatment of the plant material with a bacteriocide, fungicide, detergent, or the like.

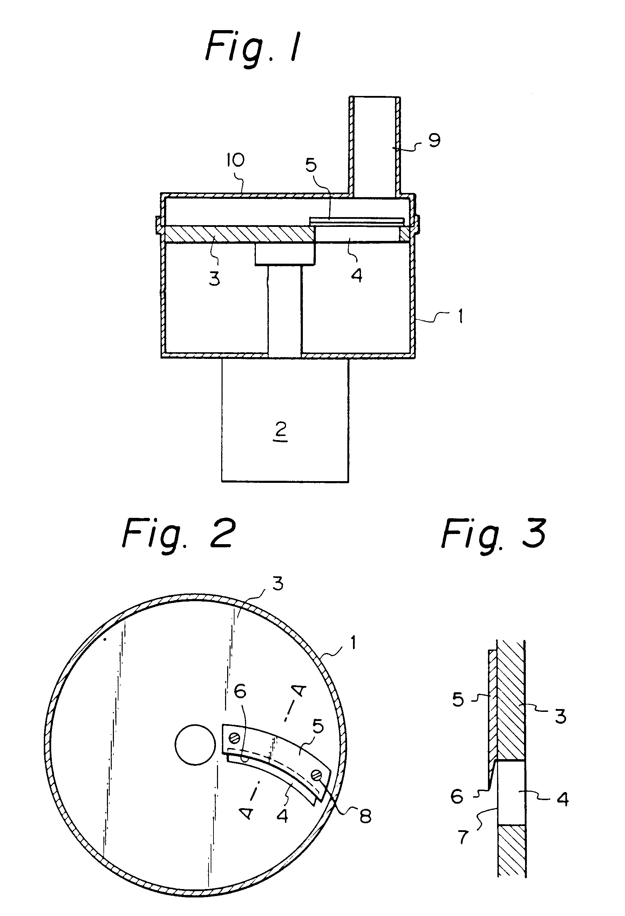

According to an embodiment of the present invention, plant sections are prepared using an apparatus shown in Figs. 1 to 6. As shown in Fig. 1, the apparatus comprises a housing 1, and a circular plate 3 driven by a drive unit 2. As shown in detail in Fig. 2 the circular plate 3 has one or more than one cutout 4 arranged at a predetermined angle relative to a tangential line of the circular plate 3. In this embodiment, this angle is approximately a right angle, i.e., the cutout is roughly radially arranged. Also as shown in Fig. 2, a blade 5 is attached to the circular plate 3 so that a cutting edge 6 thereof is positioned above the cutout, to force the plant sections cut from a plant material by the blade 5 to fall into the housing 1 through the cutout 4. A space is provided between the cutting edge 6 and a plane 7 extending from the surface of the circular plate 3 in the proximity of the blade 5. This space can be adjusted, to control the thickness of the cut plant section, by selecting the thickness of the blade 5 or by inserting a spacer (not shown) having a desired thickness between the blade 5 and the plate 3.

Plant material, especially a bulb or tuber is preferably "slip-cut" rather than "press-cut", to minimize destruction of the plant tissue. In this connection, "slip-cut" means that a cutting edge "slips" or "slides" on a cutting front of a plant material while cutting the plant material. To this end, in the embodiment shown in Fig. 2, the blade 5 and the cutout 4 have an arc-shape. When the cutting edge 6 becomes blunt or when it is necessary to re-adjust the space between the cutting edge and a plane extending along the surface of the circular plate 3, the blade 5 is changed. Accordingly, the blade 5 is removably attached to the plate 3 by, for example, screws 8. The plate 3 is driven by a drive unit, such as an electric motor, via a shaft. The plate 3 is removably attached to the top of the shaft to enable an easy recovery of cut sections accumulated in the housing 1.

Preferably, the apparatus shown in Fig. 1 further comprises a cover 10 having an inlet 9.

In the operation of the apparatus shown in Fig. 1, a speed of rotation of the circular plate 3 driven by the drive unit 2 such as an electric motor is adjusted, for example, to between 100 and 400 rpm, and the space between the cutting edge 6 and a plane 7 extending along the plate surface is adjusted to, for example, between 1 and 4 mm. The particular conditions are chosen according to a desired thickness of the cut section, the hardness of the plant material to be cut, and the like.

The apparatus shown in Fig. 1 is preferably used to prepare plant strips from the bulb of a lily or the like, or a potato tuber or the like.

The plant material is inserted into the inlet 9, and the material is then cut by the blade 5 moved by the rotation of the circular plate 3. Where a soft material such as a lily bulb is cut, the weight of the plant material per se is sufficient to press the material against the blade, and therefore, the operator can simply place the material on the circular plate. But, where a relatively hard material such as a potato tuber is to be cut, in addition to the weight of the material per se, an additional force must be applied to the material, and accordingly, a force of about 50 to 500 $g/cm^2$ is applied to the plant material. This force may be applied by placing a weight directly or via a pressing plate on the plant material, or by applying a spring force to the pressing plate, or by manually pressing down on the pressing plate.

During this operation, the plant material is cut into a plurality of strips, and the cut strips fall through the cutout 4 into the housing 1. The cut strips accumulate in the housing 1, and the cover 10 and the circular plate 3 are then removed and the cut strips recovered.

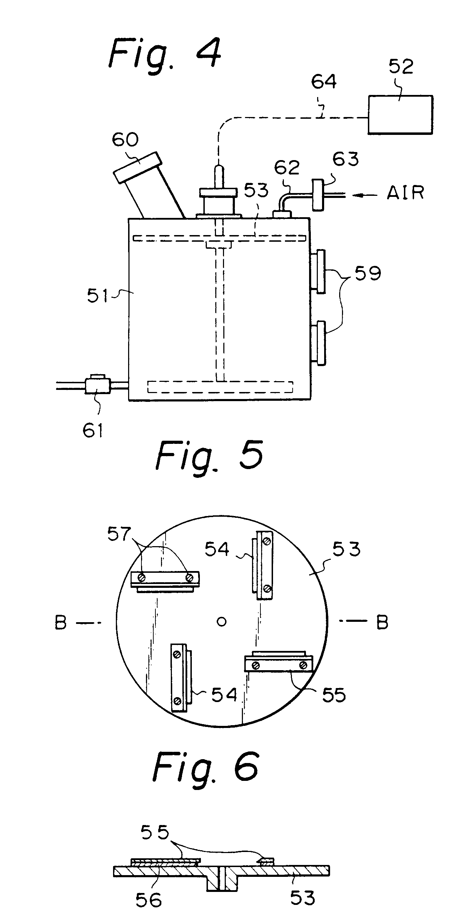

Figures 4 to 6 show a variation of the embodiment shown in Fig.1. In this variation, a circular plate 53 is provided with four cutouts 54, and accordingly, four blades 55 are attached to the circular plate 53 by screws 57. In this variation, the blades 55 have a straight cutting edge. In this case, to facilitate a "slip cut" of the plant material, the blades 55, and therefore the cutouts 54, are positioned at an angle which is not a right angle relative to a tangential line of the circular plate 53, as shown in Fig. 5.

Figure 6 shows a cross-sectional view of the plate 53 shown in Fig. 5, taken along the line B-B in Fig. 5. In the embodiment shown in Fig. 6, the blade 55 is attached to the plate 53 via a spacer 56. The purpose of the spacer 56 has been explained in detail in the description of the embodiment shown in Fig. 1. In the variation shown in Fig. 4, the circular plate 53 and the blades 55 attached thereon are contained in a housing 51. The housing 51 is provided with windows 59 through which the inside of the housing 51 can be observed, for example, to determine the amount of cut sections accumulated therein. The housing 51 is also provided with an air inlet tube 62 fitted with an air filter 63. Accordingly, by introducing sterile air through the air filter 63 and inlet tube 62, the inside of the housing 51 can be maintained in an aseptic condition during the operation, if the whole apparatus has been sterilized before the start of the operation. Note, the whole apparatus can be made from metal, and thus can be sterilized by heating, for example, steaming.

The circular plate 53 is rotated by a drive force transmitted through a flexible drive shaft 64 from a drive unit such as an electric motor 52 provided outside of the housing 51.

The apparatus shown in Fig. 4 further comprises an inlet 60 through which a plant material is fed, and an outlet 61 through which plant sections are recovered. In this variation, the inlet 60 is inclined, to facilitate the introduction of plant material while taking into consideration the presence of the flexible driving shaft 64.

The apparatus shown in Fig. 4 can be used in the same manner as described in detail for the apparatus shown in Fig. 1

Further, the apparatus shown in Fig. 4 can be preferably used to cut a plant material in a liquid medium such as water, a buffer solution, a culture medium for plant tissue culture, or the like. In this case, a liquid medium and pieces of plant material are introduced through the inlet 60, and the liquid medium containing the cut plant strips is removed through the outlet 62.

According to the present invention, a large amount of plant tissue strips which can be regenerated to plants in plant tissue culture is easily prepared without contamination and without necrosis of the plant tissue. The present method can be applied to the preparation of sections from a whole plant, or from parts of a plant, such as the flower, leaf, stem, tip, bulb, tuber, callus or the like, and the resulting sections can be cultured for the production of a useful substance or for the regeneration of the entire plant.

The present invention is now explained in detail by way of Examples.

Example 1

The apparatus shown in Fig. 1 was used for this example, and the space between the cutting edge 6 and a plane extending from a surface of the circular plate 3 was adjusted to 2 mm. The whole apparatus with cap 10 was enveloped in aluminum foil, and then autoclaved. Then, 60 virus-free bulbs of Lilium longiflorum having a diameter of 5 to 8 mm, obtained by shoot tip culture, were introduced through the inlet 9, a pressing plate (not shown) was placed on the bulb in the inlet, and a weight was placed on the pressing plate to apply a pressure of 400 g/cm² to the bulb. The cutting blades in the form of a circular plate were rotated at a speed of 350 rpm. The cut strips were cultured in a 1 ℓ mini-jar fermenter while bubbling 100% $O_2$ gas therein for 15 weeks, and 2360 bulbs having a diameter of about 3 mm were obtained.

Example 2

The same apparatus as used in Example 1 was used, but the space between the cutting edge and a plane extending from the surface of the circular plate was adjusted to 2 mm.

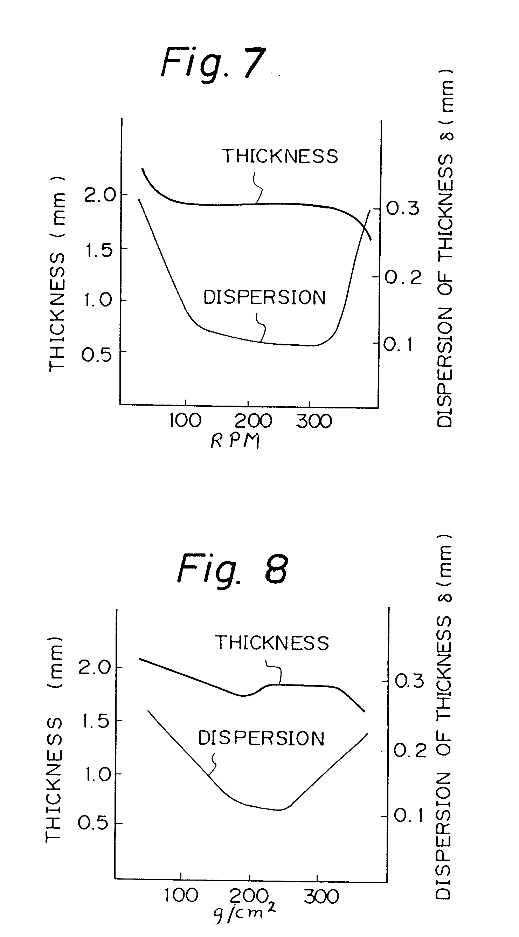

A mass of potato having a diameter of 28 mm and a length of 65 mm was introduced through the inlet 9, and a pressure of 45 g/cm² was applied to the mass. The speed of rotation was varied between 20 rpm and 400 rpm. The thickness of the cut strips and the dispersion thereof as a result of the speed of rotation are shown in Fig. 7. As seen from Fig. 7, a rotation speed of between 100 and 300 rpm provided sections having a homogeneous thickness.

Example 3

The same procedure as described in Example 2 was repeated, except that the speed of rotation was adjusted to 350 rpm, and the pressure applied to the potato mass was varied between 50 g/cm² and 400 g/cm². As shown in Fig. 8, a pressure between 200 g/cm² and 350 g/cm² provided cut strips having a homogeneous thickness.

Example 4

The apparatus shown in Fig. 4 was used to prepare cut strips of a bulb of L. longiflorum, and the results were similar to those obtained in Example 1.

**Claims**

1. A method of preparation of a plurality of plant tissue strips which can be regenerated to plants in plant tissue culture, characterised by:
   preparing a sterilised or uncontaminated plant material; and
   supplying the plant material to a cutting apparatus to prepare plant strips, wherein the cutting apparatus comprises a circular plate (3,53) having one or more than one elongated cutouts (4,54) positioned at an angle to a tangential line of the circular plate (3,53), which plate is rotated by a drive unit (2,52), and one or more than one blades (5,55) fixed to the circular plate (3,53) so that a cutting edge (6) of each blade (5,55) is positioned above each cutout (4,54) with a space between the cutting edge (6) and a plane extending from a surface of the circular plate in the proximity of the blade.

2. A method according to claim 1, wherein the blade (5) is radially arranged and has an arcuate shape, whereby the plant material is slip-cut.

3. A method according to claim 1 or 2, wherein the blade (5) is removably fixed to the circular plate by screws (8).

4. A method according to claim 1, 2 or 3, wherein the blade (55) is spaced from the circular plate by a spacer (56).

5. A method according to any preceding claim, wherein the circular plate (3,53) is arranged in a container 1,51), and both the plate (3,53) and the container (1,51) are made from a metal, whereby the whole can be sterilised by heating.

6. A method according to any preceding claim, wherein the distance between the cutting edge (6) of the blade (5) and a plane extending from a surface of the circular plate (3,53) in the proximity of the blade is between 1 and 4 mm, the plate rotates at a speed of between 100 and 400 rpm, and the plant to be cut is pressed against the blade with a pressure of between 0 and 500 $g/cm^2$, and these conditions are selected depending on the plant species, kind of plant organs and parts of the plant to be cut.

7. A method according to any preceding claim, wherein the blade is arranged at an angle smaller than a right angle relative to a tangential line of the circular plate (3,53), and the plant material is slip-cut.

8. A method of preparation of a plurality of inoculum sections for plant tissue culture, using the method of any preceding claim, wherein a liquid medium is prepared in which a sterilised or uncontaminated plant material is contained; and the plant material is cut with the apparatus inserted in a conduit through which the liquid medium flows.

9. A method according to any preceding claim, wherein a bulb or tuber of the plant is cut.

10. A method according to any one of claims 1 to 8, wherein the plant material is a plant stem having lateral buds.

## Revendications

1. Un procédé de préparation d'une pluralité de bandes de tissu végétal qui peuvent être régénérées en plantes dans la culture de tissu de plantes, caractérisé par:

la préparation d'un matériau de plante stérilisé ou non-contaminé, et

l'alimentation du matériau de plante vers un appareil de coupe pour préparer des bandes végétales, dans lequel l'appareil de coupe comprend une plaque circulaire (3, 53) présentant une ou plus d'une découpe oblongue (4, 54) positionnée suivant un angle par rapport à la tangente de la plaque circulaire (3, 53), plaque dont la rotation se fait par une unité d'entraînement (2, 52), et une ou plus d'une lame (5, 55) fixée à la plaque circulaire (3, 53), de manière qu'un bord tranchant (6) de chaque lame (5, 55) est positionné audessus de chaque découpe (4, 54) avec un espace entre le bord tranchant (6) et un plan s'étendant d'une surface de la plaque circulaire à proximité de la lame.

2. Un procédé suivant la revendication 1, dans lequel la lame (5) est disposée radialement et présente une forme arquée, le matériau de plante étant découpé par glissement.

3. Un procédé suivant la revendication 1 ou 2, dans lequel la lame (5) est fixée, de manière amovible, à la plaque circulaire par des vis (8).

4. Un procédé suivant la revendication 1, 2 ou 3, dans lequel la lame (55) est espacée de la plaque circulaire par une pièce intercalaire (56).

5. Un procédé suivant l'une ou l'autre des revendications précédentes, dans lequel la plaque circulaire (3, 53) est disposée dans un conteneur (1, 51) et tant la plaque (3, 53) que le conteneur (1, 51) sont réalisés en un métal, l'ensemble pouvant être stérilisé par chauffage.

6. Un procédé suivant l'une ou l'autre des revendications précédentes, dans lequel la distance entre le bord tranchant (6) de la lame (5) et un plan s'étendant à partir d'une surface de la plaque circulaire (3, 53) à proximité de la lame est de 1 à 4 mm, la plaque tourne à une vitesse de 100 à 400 tours-minute, et la plante à découper est poussée contre la plaque avec une pression de 0 à 500 $g/cm^2$, et ces conditions sont sélectionnées en fonction de l'espèce de plantes, du type d'organes de plante et des parties de la plante à découper.

7. Un procédé suivant l'une ou l'autre des revendications précédentes, dans lequel la lame est disposée suivant un angle plus petit qu'un angle droit par rapport a une tangente de la plaque circulaire (3, 53) et le matériau de plante est découpé par glissement.

8. Un procédé de préparation d'une pluralité de sections inoculées pour la culture de tissu de plantes, à l'aide du procédé suivant l'une ou l'autre des revendications précédentes, dans lequel est préparé un fluide liquide dans lequel est contenu un matériau de plante stérilisé ou non-contaminé, et le matériau de plante est découpé avec l'appareil introduit dans un conduit par lequel circule le fluide liquide.

9. Un procédé suivant l'une ou l'autre des revendications précédentes, dans lequel est découpé un bulbe ou un tubercule de la plante.

10. Un procédé suivant l'une ou l'autre des revendications 1 à 8, dans lequel le matériau de plante est une tige de plante présentant des boutons latéraux.

**Patentansprüche**

1. Verfahren zur Herstellung einer Vielzahl von Pflanzengewebestreifen, die bei der Pflanzengewebezüchtung zu Pflanzen regeneriert werden können, gekennzeichnet durch:
    Herstellung eines sterilisierten oder nicht verunreinigten Pflanzenmaterials; und
    Zuführung dieses Pflanzenmaterials zu einer Trennvorrichtung, um Pflanzenstreifen herzustellen, wobei diese Trennvorrichtung eine kreisförmige Platte (3, 53) mit einem oder mehr als einem Längsausschnitt (4, 54), der in einem Winkel zur Tangentiallinie der kreisförmigen Platte (3, 53) angeordnet ist, wobei diese Platte von einer Antriebseinheit (2, 52) gedreht wird, und eine oder mehr als eine Klinge (5, 55) umfaßt, die an die kreisförmige Platte (3, 53) angebracht sind, so daß eine Schnittkante (6) jeder Klinge (5, 55) mit einem Abstand zwischen der Schnittkante (6) und einer Ebene oberhalb jedes Ausschnittes (4, 54) angeordnet ist, die sich von der Oberfläche der kreisförmigen Platte in der Nähe der Klinge erstreckt.

2. Verfahren nach Anspruch 1, worin die Klinge (5) radial angeordnet ist und eine gekrümmte Form aufweist, wodurch das Pflanzenmaterial gleitend geschnitten wird.

3. Verfahren nach Anspruch 1 oder 2, worin die Klinge (5) durch Schrauben (8) abnehmbar an die kreisförmige Platte angebracht ist.

4. Verfahren nach Anspruch 1, 2 oder 3, worin die Klinge (55) durch einen Abstandhalter (56) von der kreisförmigen Platte getrennt ist.

5. Verfahren nach einem der vorstehenden Ansprüche, worin die kreisförmige Platte (3, 53) in einem Behälter (1, 51) angeordnet ist und sowohl die Platte (3, 53) als auch der Behälter (1, 51) aus Metall bestehen, wodurch das Ganze durch Erwärmen sterilisiert werden kann.

6. Verfahren nach einem der vorstehenden Ansprüche, worin der Abstand zwischen der Schnittkante (6) der Klinge (5) und der Ebene, die sich von der Oberfläche der kreisförmigen Platte (3, 53) in der Nähe der Klinge erstreckt, zwischen 1 und 4 mm beträgt und sich die Platte mit einer Geschwindigkeit von 100 bis 400 U/min dreht und die zu zerschneidende Pflanze mit einem Druck von 0 bis 500 g/cm$^2$ gegen die Klinge gedrückt wird, und diese Bedingungen in Abhängigkeit von der Pflanzenart, der Art der Pflanzenorgane und der Teile der zu trennenden Pflanze ausgewählt werden.

7. Verfahren nach einem der vorstehenden Ansprüche, worin die Klinge in einem Winkel angeordnet ist, der geringer als der rechte Winkel zur Tangentiallinie der kreisförmigen Platte (3, 53) ist, und das Pflanzenmaterial gleitend geschnitten wird.

8. Verfahren zur Herstellung einer Vielzahl von Inoculum-Abschnitten zur Pflanzengewebezüchtung unter Anwendung des Verfahrens nach einem der vorstehenden Ansprüche, wobei ein flüssiges Medium hergestellt wird, in dem das sterilisierte oder nicht verunreinigte Pflanzenmaterial enthalten ist, und dieses Pflanzenmaterial mit der Vorrichtung geschnitten wird, die in eine Leitung eingesetzt ist, durch die das flüssige Medium strömt.

9. Verfahren nach einem der vorstehenden Ansprüche, worin eine Zwiebel oder ein Knollengewächs der Pflanze geschnitten wird.

10. Verfahren nach einem der Ansprüche 1 bis 8, worin das Pflanzenmaterial ein Pflanzenstiel mit seitlichen Knospen ist.

# Fig. 1

# Fig. 2

# Fig. 3

## Fig. 4

## Fig. 5

## Fig. 6

# Fig. 7

# Fig. 8